(19) Europäisches Patentamt
European Patent Office
Office européen des brevets

(11) Numéro de publication: **0 254 642 B1**

(12) # FASCICULE DE BREVET EUROPEEN

(45) Date de publication du fascicule du brevet: **10.01.90**

(51) Int. Cl.⁴: **C07D 307/81**, A61K 31/34

(21) Numéro de dépôt: **87401703.1**

(22) Date de dépôt: **22.07.87**

(54) **Dérivés de 1,3-propanediamine, préparation et compositions pharmaceutique les contenant.**

(30) Priorité: **22.07.86 FR 8610638**

(43) Date de publication de la demande: **27.01.88 Bulletin 88/4**

(45) Mention de la délivrance du brevet: **10.01.90 Bulletin 90/2**

(84) Etats contractants désignés: **BE CH DE ES FR GB GR IT LI NL SE**

(56) Documents cités:
**FR-A- 2 193 591**

**CHEMICAL ABSTRACTS, vol. 69, no. 25, 16 décembre 1968, pages 9955-9956, résumé no. 106373p, Columbus, Ohio, US; S. TOYOSHIMA et al.: "Benzoheterocyclic derivatives. VII. Synthesis and pharmacological actions of benzofuran derivatives. I.", & YAKUGAKU ZASSHI 1968, 88(5), 503-12 000**
**REMINGTON'S PHARMACEUTICAL SCIENCES, A.R. Gennaro et al., édition 17, 1985, Mack Publishing Company, Easton, PA., US**

(73) Titulaire: **RIOM LABORATOIRES- C.E.R.M. "R.L.-CERM" - (S.A.), Route de Marsat B.P. 140, F-63203 Riom Cédex(FR)**

(72) Inventeur: **Combourieu, Michel, 14, Boulevard du Pont Rouge, F-15000 Aurillac(FR)**
Inventeur: **Landes, Marie-Paule, 20, Avenue de la Liberté, F-15000 Aurillac(FR)**
Inventeur: **Simbille, Nadine, 31, Avenue Aristide Briand, F-15000 Aurillac(FR)**
Inventeur: **Bernet, Yvon, 11, Cité des Landes Reilhac, F-15250 Jussac(FR)**

(74) Mandataire: **Hermans, Franciscus G.M. et al, Patent Department AKZO N.V. Pharma Division P.O. Box 20, NL-5340 BH Oss(NL)**

EP 0 254 642 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des dérivés de 1-3-propanediamine et plus particulièrement la N,N-diméthyl-N'-benzoyl-N'-[(2,3-dihydro) benzofuran-2-yl-méthyl] 1,3-propanediamine sous forme racémique ou chacun de ses énantiomères, ou leurs mélanges, ainsi que leurs sels pharmaceutiquement acceptables.

Ces composés répondent à la formule générale suivante :

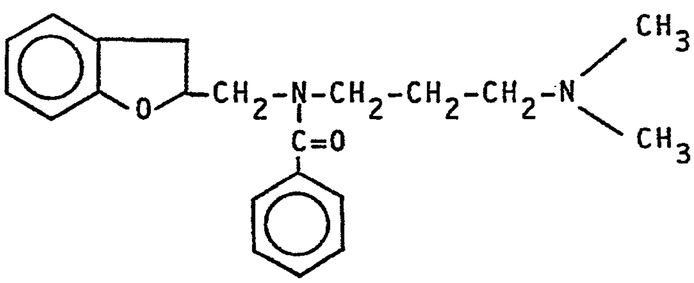

I

L'invention concerne également les procédés de préparation desdits composés et les compositions pharmaceutiques utiles en thérapeutique cardiovasculaire dans le traitement des troubles du rythme.

On connaît déjà de nombreux dérivés du benzofuranne, en particulier des dérivés substitués en 2 par une chaîne alkylamino. Par exemple, la publication de TOYOSHIMA S., HIROSE N. et Al. [Yakugaku Zasshi 88 (5), 503-512 (1968)] décrit divers dérivés de benzofuranne ; l'un de ces dérivés correspond aux composés de l'invention non benzoylés et possède notamment une activité vasocoronarodilatatrice.

Les composés de l'invention possèdent au contraire d'inté-ressantes propriétés antiarythmiques, tout en étant sans action sur les autres paramètres hémodynamiques ; ils sont notamment dépourvus d'activité vasocoronarodilatatrice.

Ces composés peuvent être préparés par des méthodes connues pour la préparation de composés analogues.

En particulier les composés de l'invention peuvent être préparés par benzoylation d'un composé de formule II

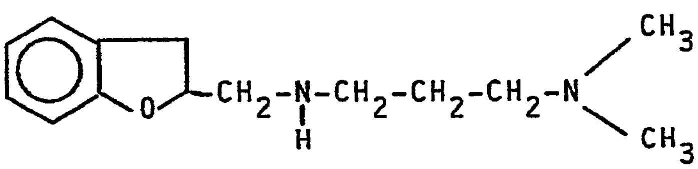

II

ou de l'un de ses sels.

Ladite benzoylation est effectuée de préférence avec un dérivé actif de benzoyle tel qu'un halogénure de benzoyle (par exemple le chlorure ou le bromure de benzoyle) ou par un ester ou un anhydre de l'acide benzoïque, mais peut aussi être effectuée avec l'acide benzoïque en présence d'un agent de déshydratation tel que le dicyclohexyl-carbodiimide ou d'autres carbodiimides.

En utilisant un dérivé actif de benzoyle, on effectue de préférence la réaction dans un solvant organique en présence d'une base telle que la triéthylamine.

Quand le composé de départ de formule II est un des énantiomères optiques, le composé résultant est évidemment l'énantiomère optique de formule I. Quand le produit de départ II est le racémique, le composé résultant de formule I est aussi le racémique.

On peut également résoudre le composé racémique de formule I par des méthodes connues pour la résolution des mélanges racémiques.

Généralement, on résout la base racémique de formule I au moyen d'un acide carboxylique optiquement actif. En particular, on peut transformer la base racémique en sel d'acide tartrique duquel il est possible de régénérer après les recristallisations habituelles, soit la base racémique, soit les isomères lévogyre ou dextrogyre selon qu'on utilise l'acide tartrique RS, RR ou SS.

La diamine de départ peut être préparée selon les procédés connus ; elle peut également être préparée à partir du 2-allyl phénol qu'on transforme en 2-[(2,3-dihydro) benzofuryl] méthanol par action d'un peracide, puis en tosylate par action du chlorure de paratoluène sulfonyl, ledit tosylate étant ensuite mis à réagir avec la 3-diméthyl amino-propylamine pour donner la N,N-diméthyl-N'-[2,3-dihydro) benzofuran-2-yl-méthyl] 1,3-propanediamine.

La préparation des composés de l'invention est illustrée plus en détails par les exemples ci-après.

Préparation de la N,N-diméthyl-N'-[(2,3-dihydro) benzofurand-2-yl-méthyl] 1,3-propanediamine.

Dans un réacteur, on a dissous 200 g (1,49 M) de 2-allyl phénol dans 2 l de chlorure de méthylène, ajouté 309 g (1,79 M) d'acide métachloroperoxybenzoïque en maintenant sous agitation à 0°C, puis à température ambiante jusqu'a disparition du 2-allyl phénol.

A la solution de 2-[2,3-dihydro)-benzofuryl] méthanol, on a ajouté 150,4 g (0,9 M) de chlorure de paratoluène sulfonyl en maintenant sous agitation 4 heures à température ambiante. Après évaporation, purification et recristallisation dans l'éthanol absolu, on a obtenu 197 g de tosylate ayant pour point de fusion F = 68 - 70°C.

On a enfin mis en suspension dans 150 ml de butanol, 23,5 g (0,075 M) du tosylate précédent et 38,6 g (0,378 M) de 3-diméthylamino propylamine et porté le mélange à reflux jusqu'à disparition du tosylate.

Après avoir évaporé le solvant puis l'excès de 3-diméthylamino propylamine, on a repris le résidu dans l'éther éthylique, lavé à l'eau, séché sur $Na_2SO_4$, filtré, évaporé à sec, puis distillé sous pression réduite et obtenu 11,4 g de la diamine attendue ayant pour point d'ébullition E = 140 - 148°C sous 50 Pa.

Exemple 1 : N,N-diméthyl-N'-benzoyl-N'-[(2,3-dihydro) benzofuran-2-yl-méthyl] 1,3-propanediamine racémique

Dans un tricol de 250 ml, on a dissous 10,6 g (0,045 M) de la diamine précédente dans 100 ml de chlorure de méthylène et ajouté 6,8 g (0,0675 M) de triéthylamine. On a ensuite introduit goutte à goutte 6,4 g (0,045 M) de chlorure de benzoyle et maintenu 1/4 d'heure sous agitation à température ambiante, puis on a rajouté 0,64 g de chlorure de benzoyle pour achever la benzoylation. On a dilué le milieu réactionnel par du chlorure de méthylène, lavé la phase organique avec une solution de NaOH à 5%, puis à l'eau et séché sur $Na_2SO_4$.

Après filtration et évaporation à sec, on a obtenu 15,2 g de base brute sous forme d'une huile épaisse qu'on a traité par l'acide tartrique.

Dans un ballon de 250 ml, on a dissous 5 g de la base brute précédemment obtenue dans un minimum d'éthanol absolu, puis ajouté 2,2 g d'acide RS tartrique en solution dans un minimum d'éthanol absolu, puis porté à - 5°C et laissé revenir à température ambiante. Après filtration et lavage à l'éthanol absolu, le précipité formé a été séché sous vide et on a obtenu 6,2 g de RS tartrate ayant pour point de fusion F = 131,2°C ± 2°C. La base régénérée de ce tartrate ne présente aucun pouvoir rotatoire.

Exemple 2: (R)-N,N-diméthyl-N'-benzoyl-N'-[(2,3-dihydro) benzofuran-2-yl-méthyl] 1,3-propanediamine

En procédant comme indiqué dans l'exemple 1, mais en partant de 5 g de base brute et de 2,2 g d'acide RR tartrique, on a obtenu après recristallisation dans l'éthanol absolu 2,8 g de RR tartrate ayant pour point de fusion F = 157 - 159°C. La base libérée de ce sel se présente sous forme d'une huile ayant pour pouvoir rotatoire

$[\alpha]_D^{20} = +42° \pm 1°$

pour une pureté optique de 97 - 98 %.

Exemple 3: (S)-N,N-diméthyl-N'-benzoyl-N'-[(2,3-dihydro) benzofuran-2-yl-méthyl] 1,3-propanediamine

En procédant comme indiqué dans l'exemple 1, mais en partant de 5,2 g de base brute et de 2,3 g d'acide SS tartrique, on a obtenu après recristallisation dans l'éthanol absolu 3,1 g de SS tartrate ayant pour point de fusion F = 156 - 158°C. La base régénérée de ce tartrate se présente sous forme d'une huile qui a pour pouvoir rotatoire $[\alpha]_D^{20} = -42° \pm 1°$

pour une pureté optique de 97 - 98 %.

L'activité antidysrythmique des composés de l'invention a été mise en évidence chez le rat anesthésié par le test de la ligature coronaire, selon le protocole de CLARK et al. [Journal of Pharmacological Methods 3, 357-368 (1980)].

Les paramètres suivants ont été comptabilisés chaque minute pendant les 30 minutes qui suivent la ligature.

Complexes ectopiques

L'évaluation est effectuée selon la cotation arbitraire suivante :

0 : rythme sinusal pur, ou présence de moins de 10 complexes ectopiques par minute.
1 : présence de moins de 50 complexes ectopiques par minute.
2 : présence de plus de 50 complexes ectopiques par minute.

Les valeurs rapportées dans le tableau ci-après représentent le pourcentage d'inhibition des complexes ectopiques par rapport au groupe contrôle.

Fibrillation ventriculaire

On note le nombre d'animaux ayant présenté un ou plusieurs épisodes de fibrillation ventriculaire d'une durée supérieure à cinq secondes. Le critère retenu pour une activité anti-fibrillante liminaire est la protection d'au moins 3 animaux sur 6.

Les valeurs rapportées dans le tableau représentent le nombre d'animaux ayant présenté une fibrillation ventriculaire sur un lot de 6 animaux.

Ces résultats sont rapportés dans le tableau ci-après, comparativement au disopyramide (dénomination commune internationale), composé connu pour son activité anti-dysrythmique. Les composés sont administrés à la dose de 1,25 $mg.kg^{-1}$ par voie intraveineuse.

| Composés | Complexes ectopiques | Fibrillation ventriculaire |
|---|---|---|
| Exemple 1 | 51% | 4 |
| Exemple 2 | 40% | 2 |
| Exemple 3 | 46% | 2 |
| Disopyramide | 0 | 3 |

Ces résultats montrent que les composés de l'invention possèdent des activités antidysrythmiques supérieures à celles du disopyramide, les isomères optiques ayant en outre une activité supérieure à celle du racémique sur la fibrillation ventriculaire.

Ces composés se sont en outre révélés posséder une toxicité réduite : aucun décès n'est observé chez la souris à 640 $mg.kg^{-1}$ par voie orale.

Ils pourront donc être utilisés en thérapeutique humaine dans le traitement des troubles du rythme.

Les composés de l'invention peuvent être administrés par voie orale ou parentérale à des doses journalières comprises entre 0,5 mg et 15 mg par kg de poids corporel selon la méthode d'administration.

En thérapeutique humaine, on utilise de préférence une dose journalière comprise entre 100 mg et 500 mg.

Associés aux excipients pharmaceutiques habituels, les composés I peuvent être mis sous formes unitaires tels que pilules, comprimés, comprimés enrobés, ou bien conditionnés en capsules. En utilisant des liquides appropriés, les composés peuvent aussi être uitilisés en préparation injectable ou en préparation orale sous forme de solutions, suspensions ou émulsions.

On pourra par exemple administrer ces composés sous forme de comprimés sécables dosés à 200 mg correspondant à la formule unitaire ci-après :

| | |
|---|---|
| Composé levogyre | 200 mg |
| Cellulose microcristalline | 70 mg |
| Amidon de blé | 10 mg |
| Polyvinylpyrrolidone | 6 mg |
| Talc | 3 mg |
| Stéarate de magnésium | 1 mg |
| | 290 mg |

Dans un mélangeur planétaire, on introduit et mélange soigneusement le principe actif, la cellulose microcristalline et l'amidon de blé, puis on mouille avec une solution de polyvinylpyrrolidone à 15 % dans l'eau et mélange jusqu'à homogénéité. La masse est ensuite granulée sur une grille de 1,60 mm et séchée en étuve à convection à 50°C. Après calibrage sur une grille de 1,25 mm, on ajoute le talc et le stéarate de magnésium, on mélange, puis on comprime avec une presse rotative pour un poids terminal de 290 mg (275,5 - 304,5) par comprimé.

**Revendications**

1. N,N-diméthyl-N'-benzoyl-N'-[(2,3-dihydro) benzofuran-2-yl-méthyl] 1,3-propanediamine de formule

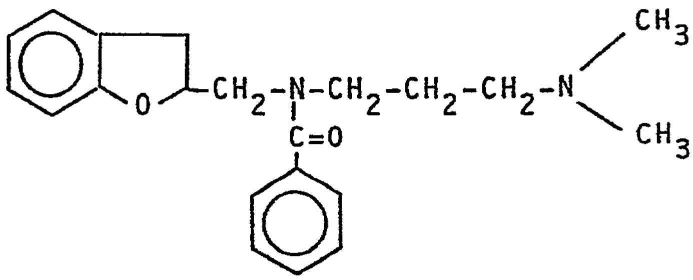

sous forme racémique, ses énantiomères et leurs mélanges et leurs sels pharmaceutiquement acceptables.

2. Composé selon la revendication 1, caractérisé en ce qu'il correspond au racémique, ou à un de ses sels pharmaceutiquement acceptables.

3. Composé selon la revendication 1, caractérisé en ce qu'il correspond à l'isomère dextrogyre,ou à un de ses sels pharmceutiquement acceptables.

4. Composé selon la revendication 1, caractérisé en ce qu'il correspond à l'isomère lévogyre, ou à un de ses sels pharmaceutiquement acceptables.

5. Procédé pour la préparation des composés selon les revendications 1 à 4, caractérisé en ce que le composé de formule

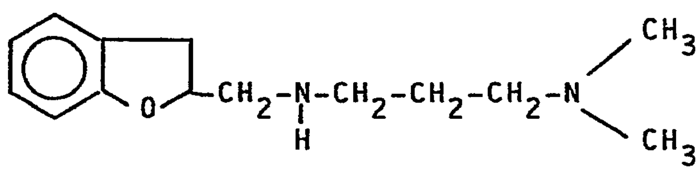

est benzoylé,
et qu'ensuite le composé ainsi obtenu, si c'est un mélange racémique, est résolu en ses énantiomères optiques et/our transformé en un sel pharmaceutiquement acceptable.

6. Procédé selon la revendication 5, caractérisé en ce qu'on traite la N,N-diméthyl-N'-[(2,3-dihydro) benzofuran-2-yl-méthyl] 1,3-propanediamine par le chlorure de benzoyle, puis la base brute obtenue par un acide tartrique RS, RR ou SS pour obtenir un tartrate duquel on libère, après recristallisation, l'amine.

7. Composition pharmaceutique, caractérisée en ce qu'elle contient, à titre de principe actif, un des composés selon l'une des revendications 1 à 4.

**Claims**

1. N,N-Dimethyl-N'-benzoyl-N'-[(2,3-dihydro)benzofuran-2-ylmethyl]-1,3-propanediamine of the formula

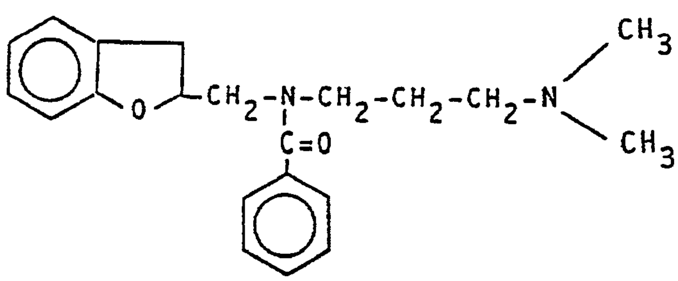

in the racemic form, its enantiomers and mixtures of them and their pharmaceutically acceptable salts.

2. Compound according to claim 1, characterized in that it corresponds to the racemic mixture, or to one of its pharmaceutically acceptable salts.

3. Compound according to claim 1, characterized in that it corresponds to the dextrorotatory isomer, or to one of its pharmaceutically acceptable salts.

4. Compound according to claim 1, characterized in that it corresponds to the laevorotatory isomer, or to one of its pharmaceutically acceptable salts.

5. Process for the preparation of compounds according to claims 1 to 4, characterized in that the compound of formula

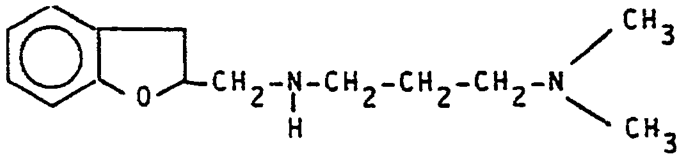

is benzoylated, and that then the compound thus obtained, if it is a racemic mixture, is resolved into its optical enantiomers and/or converted to a pharmaceutically acceptable salt.

6. Process according to claim 5, characterized in that N,N-dimethyl-N′-[(2,3-dihydro)benzofuran-2-yl-methyl]-1,3-propanediamine is treated with benzoyl chloride, then the crude base obtained is treated with a (RS)-, (RR)- or (SS)-tartaric acid to obtain a tartrate from which the amine is liberated after recrystallization.

7. Pharmaceutical composition, characterized in that it contains, as active principle, one of the compounds according to one of claims 1 to 4.

**Patentansprüche**

1. N,N-Dimethyl-N′-benzoyl-N′-[(2,3-dihydro)-benzfuran-2-yl-methyl]-1,3-propandiamin der Formel

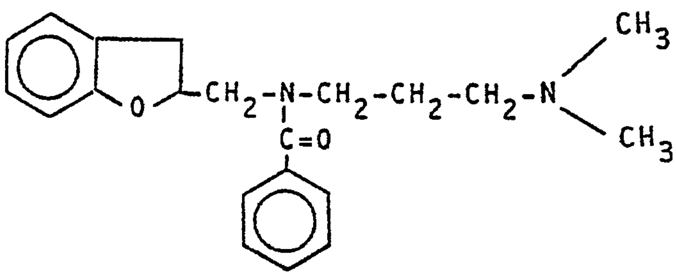

in der racemischen Form, dessen Enantiomere und dessen Gemische sowie dessen pharmazeutisch unbedenkliche Salze.

2. Verbindung nach Anspruch 1, dadurch gekennzeichnet, daß sie dem Racemat oder einem von dessen pharmazeutisch unbedenklichen Salzen entspricht.

3. Verbindung nach Anspruch 1, dadurch gekennzeichnet, daß sie dem rechtsdrehenden Isomeren oder einem von dessen pharmazeutisch unbedenklichen Salzen entspricht.

4. Verbindung nach Anspruch 1, dadurch gekennzeichnet, daß sie dem linksdrehenden Isomeren oder einem von dessen pharmazeutisch unbedenklichen Salzen entspricht.

5. Verfahren zur Herstellung von Verbindungen nach Ansprüchen 1 bis 4, dadurch gekennzeichnet, daß man die Verbindung der Formel

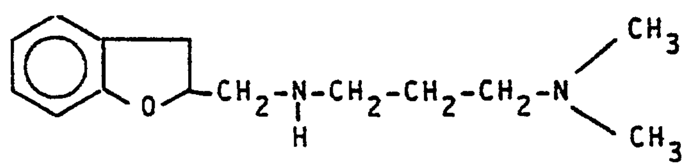

benzoyliert und anschließend die so erhaltene Verbindung, falls sie ein racemisches Gemisch ist, in deren optische Enantiomere auftrennt und/oder in ein pharmazeutisch unbedenkliches Salz überführt.

6. Verfahren nach Anspruch 5, dadurch gekennzeichnet, daß man N,N-Dimethyl-N′-[(2,3-dihydro)-benzfuran-2-yl-methyl]-1,3-propandiamin mit Benzoylchlorid und anschließend die erhaltene rohe Base mit RS-, RR- oder SS-Weinsäure behandelt, um ein Tartrat zu erhalten, aus dem man nach Umkristallisation das Amin freisetzt.

7. Pharmazeutische Zusammensetzung, dadurch gekennzeichnet, daß sie als Wirkstoff eine der Verbindungen nach einem der Ansprüche 1 bis 4 enthält.